(19) Europäisches Patentamt
European Patent Office
Office européen des brevets

(11) Publication number: **0 112 266**
**B1**

(12) **EUROPEAN PATENT SPECIFICATION**

(45) Date of publication of patent specification: **09.03.88**

(51) Int. Cl.$^4$: **C 07 D 451/06,** A 61 K 31/46

(21) Application number: **83440062.4**

(22) Date of filing: **06.12.83**

(54) **Substituted tropyl and pseudotropyl benzoates and their use in migraine treatment.**

(30) Priority: **10.12.82 GB 8235296**

(43) Date of publication of application:
**27.06.84 Bulletin 84/26**

(45) Publication of the grant of the patent:
**09.03.88 Bulletin 88/10**

(84) Designated Contracting States:
**AT BE CH DE FR IT LI LU NL SE**

(56) References cited:
**FR-A-1 161 325**

**CHEMICAL ABSTRACTS, vol. 78, 1973, page 22, no. 119200r, Columbus, Ohio, US; S. GHOSAL et al.: "Desmodium alkaloids. II. Chemical and pharmacological evaluation of D. gangeticum" & PLANTA MED. 1972, 22(4), 434-40**

(73) Proprietor: **MERRELL DOW FRANCE ET CIE**
**16 Rue d'Ankara**
**F-67084 Strasbourg (FR)**

(72) Inventor: **Fozard, John R.**
**22 rue Vermeer**
**F-67200 Strasbourg-Elsau (FR)**
Inventor: **Gittos, Maurice W.**
**16 rue Andre Malraux Plobsheim**
**F-67400 Illkirch-Graffenstaden (FR)**

(74) Representative: **Vossius & Partner**
**Siebertstrasse 4 P.O. Box 86 07 67**
**D-8000 München 86 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 112 266 B1**

Courier Press, Leamington Spa, England.

**Description**

The invention relates to the treatment of migraine with certain substituted tropyl and pseudotropyl benzoates and provides pharmaceutical compositions comprising said compounds and methods of treating migraine using said compounds. Further, the invention also provides said compounds for use in treating migraine and, when said compounds are novel, it provides said novel compounds *per se*.

Acute attacks of migraine are usually treated with a peripheral vasoconstrictor, such as ergotamine, which may be co-administered with caffeine, and dihydroergotamine; an antipyretic analgesic, such as acetylsalicylic acid or p-acetylaminophenol; and/or an anti-emetic such a cyclizine, metoclopramide and thiethylperazine. It has also been reported (J. B. Hughes; Med. J. Aust. 2, No. 17, 580, 1977) that immediate relief of acute migraine attack can be obtained by slow intravenous injection of metoclopramide (10 mg).

It is believed that 5-hydroxytryptamine (5-HT) is the naturally occurring substance most likely to play a role in the pathophysiology of migraine. Increased amounts of 5-HT and its metabolite 5-hydroxyindoleacetic acid are excreted in the urine during most attacks. Further, plasma and platelet 5-HT concentrations fall rapidly at the onset of an attack and remain low whilst the headache persists. Morever, attacks of migraine have been clearly associated with periods of thrombocytopaenia in certain patients. It has been proposed that compounds which block the activity of 5-HT would be of use in the treatment of migraine (J. R. Fozard, International Headache Congress 1980) reported in Advances in Neurology, Vol 33, Raven Press, New York 1982).

The known migraine prophylactic drugs methysergide, propranolol, amitriptyline, and chlorpromazine have widely different pharmacological activities but are all 5-HT D-receptor antagonists at the doses used clinically for the treatment of migraine. Metoclopramide is a potent 5-HT M-receptor antagonist and it has been proposed (J. R. Fozard *supra*) that blockade of the M-receptor present on afferent sensory neurones affords symptomatic relief in an acute migraine attack.

The potency as 5-HT M-receptor antagonist of (−) cocaine and some related compounds, include benzoyl pseudotropine (i.e. pseudotropyl benzoate) and benzoyl tropine (i.e. tropyl benzoate), has been reported (J. R. Fozard *et al*, Eur. J. Pharmacol., 59(1979), 195—210) but, with the exceptions of nor(−)cocaine and and benzoyl tropine, none are as potent as metoclopramide. The $pA_2$ values reported for nor(−)cocaine, benzoyl tropine, and benzoyl pseudotropine are 7.7, 7.2 and 7.0 respectively, whilst the $pA_2$ 5-HT value determined for metoclopramide by the same procedure is 7.2 (J. R. Fozard *et al.* Eur. J. Pharmacol., 49(1978), 109—112).

It has been reported in EP—A—0067770 that substitution of tropyl benzoate with alkyl, alkoxy or halogen in the 3,4 and 5 or 3,4, and 5 positions of the benzene ring surprisingly enhances its potency as a 5-HT M-receptor antagonist. Tests conducted with tropyl-4-chlorobenzoate ($pA_2$ 7.0), tropyl-4-methylbenzoate ($pA_2$ 7.8), tropyl-3,4-dichlorobenzoate ($pA_2$ 7.8) and tropyl-3,4-dimethoxybenzoate ($pA_2$ 7.2), indicated that corresponding substitutions elsewhere in the benzene ring would not provide the same order of increase in potency. Further, tests conducted with pseudotropyl-3,5-dimethoxybenzoate ($pA_2$ 6.6) and pseudotropyl-3,4,5-trimethoxybenzoate ($pA_2$ 5.7) indicated that corresponding substitutions in the benzene ring of pseudotropyl benzoate actually would reduce its potency as a 5-HT M-receptor antagonist. However, it has now surprisingly been found that substitution by $C_1$—$C_4$ alkoxy or chlorine in at least the 2 position of the benzene ring of tropyl benzoate and pseudotropyl benzoate does substantially enhance potency as a 5-HT M-receptor antagonist.

With the exceptions of tropyl 2-chlorobenzoate and tropyl 2,4-, 2,5- and 2,6-dichlorobenzoate, the tropyl benzoate derivatives of Formula I are believed to be novel compounds. All the pseudotropyl benzoates of Formula I are believed to be novel compounds. The said tropyl 2-chlorobenzoate and tropyl-dichlorobenzoates are disclosed in Schultz *et al* (Pharm. Zig. 1972, 117 (40), 1455—6) and stated to exhibit spasmolytic- inducing activity on isolated guinea pig ileum but no pharmacological use has been reported for the compounds.

According to a first aspect of the invention there are provided novel tropyl or pseudotropyl benzoate derivatives having the following general Formula I

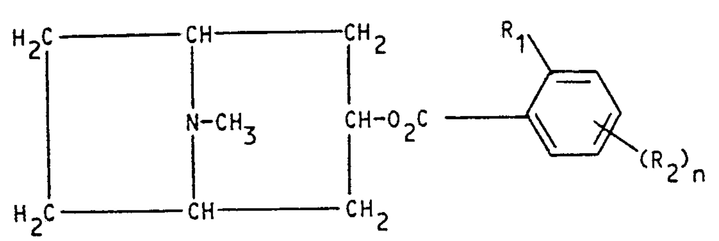

(I)

wherein:

$n$ represents 0 or an integer from 1 to 4;

$R_1$ represents $C_1$—$C_4$ alkoxy or chlorine provided that $R_1$ is not chlorine when $n$ is 0;

$R_2$ represents $C_1$—$C_4$ alkyl, $C_1$—$C_4$ alkoxy or halogen, provided that $R_2$ is not chlorine in the 4, 5, or 6 position when $R_1$ is chlorine and $n$ is 1, and that $R_2$ is the same as $R_1$ when $n$ is 2, 3, or 4;

or a pharmaceutically acceptable salt thereof.

According to a second aspect of the invention there are provided pharmaceutical compositions in unit dose form comprising a compound of general formula I with the definition of the residues according to the first aspect of the invention in admixture or otherwise associated with a pharmaceutically acceptable diluent or carrier and containing 0.5 to 100 mg, preferably 3 to 30 mg per unit dose.

According to a third aspect of the invention, there are provided for use in the treatment of migraine and other vascular headaches a tropyl or pseudotropyl benzoate derivative of the following general formula I:—

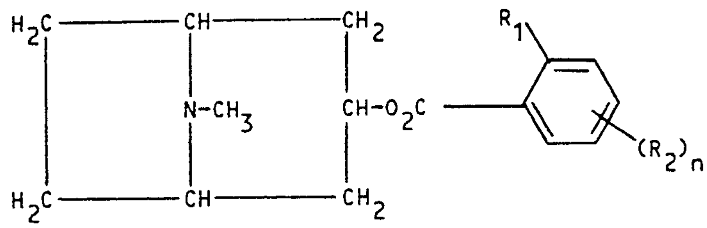

(I)

wherein:—

$n$ represents 0 or an integer from 1 to 4;

$R_1$ represents $C_1$—$C_4$ alkoxy or chlorine; and

$R_2$ represents $C_1$—$C_4$ alkyl, halogen or $C_1$—$C_4$ alkoxy, provided that $R_2$ is the same as $R_1$ when $n$ is 2, 3, or 4;

or a pharmaceutically acceptable salt thereof.

According to a fourth aspect of the invention there are provided pharmaceutical compositions in unit dose form for the effective relief of migraine, comprising a compound of general Formula I with the definition of the residues according to the third aspect of the invention in admixture or otherwise associated with a pharmaceutically acceptable diluent or carrier and containing 0.5 to 100 mg per unit dose. Usually, said compositions will contain 1 to 50 mg, especially 3 to 30 mg, per unit dose.

For treating migraine an effective migraine relieving amount of a compound of Formula 1 is administered to a patient suffering migraine. Said amount usually will be in the range 0.01 mg/kg to 10 mg/kg, especially 0.03 mg/kg to 3.0 mg/kg. It is also contemplated that the compounds of Formula I can be used in the prophylaxis of migraine by administering to a patient at risk of migraine an effective migraine-prophylatic amount of the compound.

When the bicyclic ring of a compound of Formula I is in the endo configuration, the compund is a tropyl benzoate and, when it is in the exo configuration, the compound is a pseudotropyl benzoate. Presently the tropyl benzoates are preferred over the pseudotropyl benzoates.

The compounds of general Formula I have the benzoyloxy moiety substituted by $C_1$—$C_4$ alkoxy or chlorine in at least the 2 position and optionally in other positions. In particular $R_1$ represents $C_1$—$C_4$ alkoxy or chlorine; $R_2$ represents $C_1$—$C_4$ alkyl, $C_1$—$C_4$ alkoxy or halogen but is the same as $R_1$ when there are two or three substituents represented by $R_2$; and $n$ is 0 or an integer up to 4 preferably 0, 1 or 2.

In general Formula I, the phenyl ring can be substituted as shown in the following Table I:—

TABLE I

| Position | 2 | 3 | 4 | 5 | 6 |
|---|---|---|---|---|---|
| | $R_1$ | H | H | H | H |
| | $R_1$ | $R_2$ | H | H | H |
| | $R_1$ | H | $R_2$ | H | H |
| | $R_1$ | H | H | $R_2$ | H |
| | $R_1$ | H | H | H | $R_2$ |
| | $R_1$ | $R_1$ | $R_1$ | H | H |
| | $R_1$ | $R_1$ | H | $R_1$ | H |
| | $R_1$ | $R_1$ | H | H | $R_1$ |
| | $R_1$ | H | $R_1$ | $R_1$ | H |
| | $R_1$ | H | $R_1$ | H | $R_1$ |
| | $R_1$ | $R_1$ | $R_1$ | $R_1$ | H |
| | $R_1$ | $R_1$ | $R_1$ | H | $R_1$ |
| | $R_1$ | $R_1$ | H | $R_1$ | $R_1$ |
| | $R_1$ | $R_1$ | $R_1$ | $R_1$ | $R_1$ |

In the table above, $R_1$ and $R_2$ are as defined in connection with Formula I.

Examples of $C_1$—$C_4$ alkoxy groups which can be represented by $R_1$ and $R_2$ are methoxy, ethoxy, *n*-propoxy, *n*-butoxy, and *iso*-propoxy, with ethoxy and especially, methoxy being preferred.

Examples of $C_1$—$C_4$ alkyl groups which can be represented by $R_2$ are methyl, ethyl, *n*-propyl, *n*-butyl and *iso*-propyl with ethyl and especially methyl being preferred.

The halogens which can be represented by $R_1$ and $R_2$ are bromine, chlorine, fluorine and iodine with bromine, fluorine and, especially, chlorine being preferred.

One preferred class of compounds of the invention are those pseudotropyl benzoates and tropyl benzoates of Formula I in which *n* is 0 or an integer from 1 to 4, $R_1$ represents chlorine and $R_2$ represents $C_1$—$C_4$ alkyl or chlorine provided that $R_2$ is chlorine when *n* is 2, 3, or 4.

In a presently particularly preferred embodiment of the invention, the compounds are those pseudotropyl benzoates and, especially, tropyl benzoates of Formula I in which $R_1$ and, if present, $R_2$ represent chlorine especially at the 2; 2, 3; 2, 4; or 2, 5; positions. The said especially preferred chlorine substituted compounds are the following:—

tropyl-2-chlorobenzoate,
tropyl-2,3-dichlorobenzoate,
tropyl-2,4-dichlorobenzoate,
tropyl-2,5-dichlorobenzoate,
pseudotropyl-2-chlorobenzoate,
pseudotropyl-2,3-dichlorobenzoate,
pseudotropyl-2,4-dichlorobenzoate,
pseudotropyl-2,5-dichlorobenzoate

In addition, to the preferred compounds specified above, the following are illustrative compounds of Formula I:—

tropyl 2-chloro-3-methylbenzoate;
tropyl 2-chloro-4-methylbenzoate;
tropyl 2-chloro-5-methylbenzoate;
tropyl 2,3,5-trichlorobenzoate;
tropyl 2,3,4,5-tetrachlorobenzoate;
tropyl 2,3,4,5,6-pentachlorobenzoate;

tropyl 2-methoxybenzoate;
tropyl 2,5-dimethoxybenzoate;
tropyl 2-chloro-5-methoxybenzoate;
pseudotropyl 2,3,5-trichlorobenzoate;
pseudotropyl 2-chloro-4-methylbenzoate;

The compounds of Formula I block the M-receptors for 5-hydroxytryptamine (5-HT) on afferent sensory neurones, certain of which subserve the transmission of pain. As explained above, the blocking of such M-receptors is believed to be a mechanism by which the symptoms of migraine can be relieved. Accordingly, the compounds of Formula I are useful in the treatment of migraine when administered in amounts sufficient to effectively block the said M-receptors.

The activity of the compounds against 5-HT can be assessed by determining their $pA_2$ values in the isolated rabbit heart as described by Fozard *et al* Europ. J. Pharmacol. *59*, 195—210 (1979). In the method described the molar concentration of antagonist which reduces the effects of twice the ED50 of 5-HT to that of ED50 in the absence of antagonist is determined. The $pA_2$ value is the negative logarithm of said molar concentrations. In general terms, the highter the $pA_2$ value the more potent is the compound.

It is believed that the compounds of Formula I show in this test a potency as 5-HT M-receptor antagonists at least an order greater than that of troplybenzoate or pseudotropyl benzoate.

The activity of the compounds against 5-HT can be assessed *in vivo* by measurement of the effect of the compound of the Von Bezold-Jarisch Reflex induced by 5-HT injected intraveneously into the rat (see Paintal A. S., Physiol. Rev. *53* 159—227, 1973). The transient cardiac slowing arises from an increased efferent vagus activity arising from stimulation by 5-HT of sensory afferent fibres in and around the heart.

The compounds of Formula I are believed to be highly selective in their action against 5-HT M-receptor. Their potency against other 5-HT receptors and other spasmogens, in particular oxytocin, acetylcholine, histamine and calcium, is believed to be at least two orders lower than that against 5-HT M-receptors. Accordingly, their use in the treatment of migraine should be without any side effects.

The compounds of Formula I can be administered in various manners to achieve the desired effect. The compounds can be administered alone or in the form of pharmaceutical preparations to the patient being treated either orally or parenterally, for example, subcutaneously or intraveneously. The amount of compound administered will vary and can be any effective migraine-relieving amount. Depending upon the patient and the mode of administration, the quantity of compound administered may vary over a wide range to provide from about 0.01 mg/kg to about 10 mg/kg, usually 0.03 to 3.0 mg/kg, of body weight of the patient per dose. Unit doses of these compounds can contain, for example, from about 0.5 mg to 100 mg, usually 1 to 50 mg and preferably 3 to 30 mg, of the compound and may be administered, for example, from 1 to 4 times daily.

The term "unit dosage form" is used herein to mean a single or multiple dose form containing a quantity of the active ingredient in admixture with or otherwise in association with the diluent or carrier, said quantity being such that one or more predetermined units are normally required for a single therapeutic administration. In the case of multiple dose forms such as liquids or scored tablets, said predetermined unit will be one fraction, such as a 5 ml (teaspoon) quantity of a liquid or a half or quarter of a scored tablet, of the multiple dose form.

In the composition aspect of the invention there are provided pharmaceutical formulations in which form the active compounds of the invention will normally be utilized. Such formulations are prepared in a manner well known *per se* in the pharmaceutical art and usually comprise at least one active compound of the invention in admixture or otherwise in association with a pharmaceutically acceptable carrier or diluent therefor. For making those formulations the active ingredient will usually be mixed with a carrier, or diluted by a diluent, or enclosed or encapsulated in a capsule, sachet, cachet, paper or other container. A carrier or diluent may be solid, semi-solid or liquid material which serves as a vehicle, excipient or medium for the active ingredient. Suitable carriers or diluents are well known *per se*.

The formulations of the invention may be adapted for enteral or parenteral use and may be administered to the patient in the form of tablets, capsules, suppositories, solutions, suspensions or the like.

In the specific examples included hereinbelow illustrative examples of suitable pharmaceutical formulations are described.

The derivatives of Formula I can be used in migraine therapy with antimigraine drugs having different modes of action. Such drugs include those used prophylactically, such as barbiturates, diazepam, chlorpromazine, amitriptyline, propranolol, methysergide, pizotifen, cyproheptadine, ddihydroergotamine and clonidine, and those used in the acute attack, such as vasoconstrictor agents, e.g., ergotamine and dihydroergotamine, analgaesic/antiinflammatory agents, e.g., asprin, paracetamol and indomethacin, or anti-nauseants, e.g., cyclizine, metoclopramide, and triethylperazine (see Fozard, J. R. J. Pharm. Pharmacol *27*, 297—321 (1975); Saper, J. R. J. Amer. Med. Assoc. *239*, 480—484 (1978); Fozard, J. R., supra.) As an example, compounds of general Formula I could be beneficial in combination with asprin 300—1200 mg or methsergide, 2—6 mg given daily.

The compounds of general Formula I can be prepared in manner known *per se* from tropine or pseudotropine and an acid halide of the following general Formula IV:—

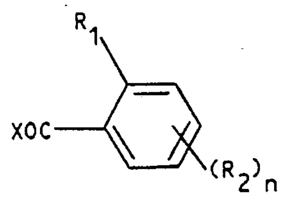

(IV)

wherein $R_1$ and $R_2$ are as defined in connection with Formula I, and X represents halogen, especially chlorine.

When preparing tropyl benzoate derivatives, the reaction usually will be carried out in the absence of a solvent by heating, at for example a temperature in the range 140° to 160°C, the acid halide with a hydrohalide salt of tropine whilst stirring. Hydrogen halide is evolved and the mixture first becomes liquid but subsequently becomes solid. Heating is continued for about 15 mins after solidification and the mixture is then cooled and added to water. The product is the hydrohalide of the compound of Formula I and the free base can be obtained by addition of aqueous base, such as sodium or potassium carbonate, which does not hydrolyse the ester, to render the aqueous product solution alkaline and subsequent extraction of the free base with a suitable organic solvent such as, for example, diethylether, ethylacetate and methylene chloride. The organic solution is subsequently evaporated and the residue recrystallized from, for example, aqueous methanol.

When preparing pseudotropyl benzoate derivatives, the reaction usually will be carried out by stirring the acid halide and pseudotropine in an aprotic solvent, preferably methylene chloride or acetonitrile, at ambient temperature. The solvent is evaporated off, usually under reduced pressure, after about 2 to 16 hours and water added to the residue, followed by aqueous base, such as sodium or potassium carbonate, which does not hydrolyse the ester, to render the aqueous product solution alkaline. Subsequently desired free base is extracted with a suitable organic solvent such as, for example, diethyl ether, ethylacetate and methylene chloride. The organic solution is then washed with water to remove excess pseudotropine and dried. The organic solvent is evaporated off and the free base recrystallized from, for example, aqueous methanol. Alternatively, the crude free base can be converted into an acid addition salt, preferably hydrochloride, by addition of an ethereal solution of the acid. The acid salt is recrystallized from, for example ethanol or isopropanol.

As mentioned previously, the compounds of Formula I can be used in the form of their pharmaceutically acceptable acid addition salts.

The pharmaceutically acceptable acid addition salts can be non-toxic addition salts with suitable acids, such as those with inorganic acids, for example hydrochloric, hydrobromic, nitric sulfuric or phosphoric acids, or with organic acids, such as organic carboxylic acids, for example, glycollic, maleic, hydroxymaleic, malic, tartaric, citric, salicylic, *o*-acetyloxybenzoic, nicotinic or isonicotinic, or organic sulphonic acids, for example methane sulphonic, ethane sulphonic, 2-hydroxyethane sulphonic, toluene-*p*-sulphonic, or naphthalene-2-sulphonic acids.

Apart from pharmaceutically acceptable acid addition salts, other acid addition salts, such as for example, those with picric or oxalic acid, may be serve as intermediates in the purification of the compounds or in the preparation of other, for example, pharmaceutically acceptable, acid addition salts, or are useful for indentification or characterisation of the bases.

An acid addition salt may be converted into the free compound according to known methods, for example, by treating it with a base, such as with a metal hydroxide or alkoxide, for example an alkali or alkaline earth metal hydroxide, for example, lithium hydroxide, sodium hydroxide, potassium hydroxide or calcium hydroxide; with a metal carbonate, such as an alkali metal or an alkaline eath metal carbonate or hydrogen carbonate, for example, sodium, potassium or calcium carbonate or hydrogen carbonate; with trialkylamine; or with an anion exchange resin.

An acid addition salt may also be converted into another acid addition salt according to known methods; for example, a salt with an inorganic acid may be treated with a metal salt, for example a sodium, barium or silver salt, or an acid in a suitable diluent, in which a resulting inorganic salt is insoluble and is thus removed from the reaction medium. Acid addition salt may also be converted into another acid addition salt by treatment with an anion exchange preparation.

The invention is illustrated in the following non-limiting Examples.

## Example 1

Tropyl-2,4-dichlorobenzoate (i.e. 2,4-dichlorobenzoic acid endo-8-methyl-8-azabicyclo[3,2,1]oct-3-yl ester) (formula I, endo, n = 1, $R_1 = R_2$ = chlorine)

A stirred mixture of tropine hydrochloride (1.76g) and 2,4-dichlorobenzoyl chloride (2.05g) is heated at 130—140°C for 30 minutes during which time the mixture liquifies, evolves hydrogen chloride gas and resolidifies. A solution of the cooled solid in water is basified with a solution of potassium carbonate and the base extracted with ethyl acetate. The ethyl acetate solution is washed several times with water, dried over magnesium sulphate, and evaporated to give the free base which is converted to the hydrochloride by the addition of ethereal hydrogen chloride. Recrystallization of the precipitated solid from ethanol gives tropyl 2,4-dichlorobenzoate hydrochloride.

Example 2

Pseudotropyl 2,4-dichlorobenzoate (i.e. 2,4-dichlorobenzoic acid exo-8-methyl-8-azabicyclo[3,2,1]oct-3-yl ester) (formula I, exo, n = 1, $R_1$ = $R_2$ = chlorine

Pseudotropine (1.41g) and 2,4 dichlorobenzoylchloride (2.05g) in methylene chloride (50 ml) is stirred at ambient temperature for 3 hours. The solvent is evaporated off under reduced pressure and the residue treated with water (50 ml) and saturated aqueous potassium carbonate solution (5 ml) is added. The liberated oil is extracted into diethylether, the ethereal solution washed several times with water and then dried over anhydrous magnesium sulfate. Distillation of the dried ethereal solution gives a residue which is treated with anhydrous diethyl ether and ethereal hydrogen chloride. Crystallizatioon of the precipitate from ethanol affords pseudotropyl-2,4-dichlorobenzoate hydrochloride.

In the following Examples relating to pharmaceutical compositions, the term "active compound" is used to indicate the compund tropyl-2,4-dichlorobenzoate. This compound may be replaced in these compositions by any other compound of Formula I, for example by pseudotropyl-2,4-dichlorobenzoate. Adjustments in the amount of medicament may be necessary or desirable depending upon the degree of activity of the medicament as is well known in the art.

Example 3

An illustrative composition for hard gelatin capsules is as follows:—

| | |
|---|---|
| (a) active compound | 5 mg |
| (b) talc | 5 mg |
| (c) lactose | 90 mg |

The formulation is prepared by passinng the dry powders of (a) and (b) through a fine mesh screen and mixing them well. The powder is then filled into hard gelatin capsules at a net fill of 100 mg per capsule.

Example 4

An illustrative composition for tablets is as follows:—

| | |
|---|---|
| (a) active compound | 5 mg |
| (b) starch | 43 mg |
| (c) lactose | 50 mg |
| (d)magnesium stearate | 2 mg |

The granulation obtained upon mixing the lactose with the compound (a) and part of the starch and granulating with starch paste is dried, screened, and mixed with the magnesium stearate. The mixture is compressed into tables weighing 100 mg each.

Example 5

An illustrative composition for an injectable suspension is the following 1 ml amuple for an intramuscular injection:—

| | Weight per cent |
|---|---|
| (a) active compound | 0.01 |
| (b) polyvinylpyrrolidone | 0.5 |
| (c) lecithin | 0.25 |
| (d) Water for injection to make | 100.0 |

The material (a)—(d) are mixed, homogenized, and filled into 1 ml ampules which are sealed and autoclaved 20 minutes at 121°C. Each ample contains 1.0 mg per ml of compound (a).

Example 6

| | mg/suppository |
|---|---|
| Active Compound | 5 |
| Oil of Theobroma (cocoa butter) | 995 |

The medicament is powdered and passed through a B.S. No. 100 Sieve and triturated with molten oil of theobroma at 45°C to form a smooth suspension. The mixture is well stirred and poured into moulds each of nominal 1G capacity, to produce suppositories..

**Claims for the Contracting States: BE CH DE FR IT LI LU NL SE**

1. A tropyl or pseudotropyl benzoate derivative having the following general formula I

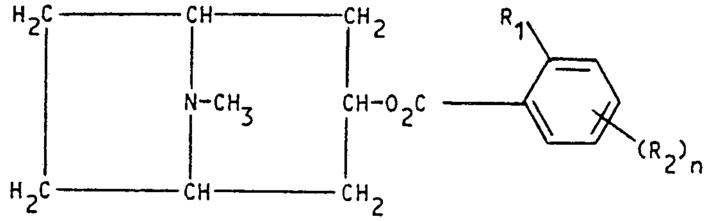

(I)

wherein

$n$ represents 0 or an integer from 1 to 4;

$R_1$ represents $C_1$—$C_4$ alkoxy or chlorine provided that $R_1$ is not chlorine when $n$ is 0;

$R_2$ represents $C_1$—$C_4$ alkyl, $C_1$—$C_4$ alkoxy or halogen, provided that $R_2$ is not chlorine in the 4, 5 or 6 position when $R_1$ is chlorine and $n$ is 1, and that $R_2$ is the same as $R_1$ when $n$ is 2, 3 or 4;

or a pharmaceutically acceptable salt thereof.

2. A compound according to Claim 1 wherein $R_1$ is methoxy or chlorine and $R_2$ represents methyl, methoxy or chlorine.

3. A compound according to Claim 1 wherein $R_1$ and $R_2$ are both chlorine.

4. A compound according to Claim 1 wherein $n$ is 1 and the phenyl group is substituted in the 2,3; 2,4 or 2,5 position.

5. A process for preparing a compound of Claim 1 which comprises reacting tropine or pseudotropine with an acid halide having the formula IV

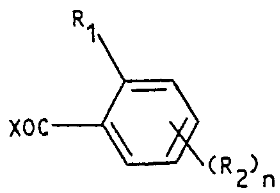

(IV)

wherein $R_1$, $R_2$ and $n$ are as defined in Claim 1 and X represents halogen.

6. A process according to Claim 5 wherein the hydrohalide salt of tropine is heated with the acid halide in the absence of a solvent.

7. A process according to Claim 5 wherein pseudotropine is stirred with an acid halide in an aprotic solvent at ambient temperature.

8. A pharmaceutical composition comprising an amouunt of 0.5 to 100 mg per unit dose of a tropyl or pseudotropyl benzoate derivative having the formula I according to claim 1 or a pharmaceutically acceptable salt thereof, in admixture or otherwise associated with a pharmaceutically acceptable diluent or carrier.

9. A composition according to Claim 8 containing from 3 to 30 mg of said compound per unit dose.

10. A tropyl or pseudotropyl benzoate derivative having the following general formula I

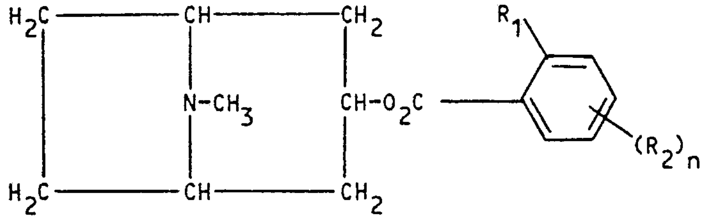

(I)

wherein:

$n$ represents 0 or an integer from 1 to 4;

$R_1$ represents $C_1$—$C_4$ alkoxy or chlorine;

$R_2$ represents $C_1$—$C_4$ alkyl, $C_1$—$C_4$ alkoxy or halogen provided that $R_2$ is the same as $R_1$ when $n$ is 2, 3, or 4;

or a pharmaceutically acceptable salt thereof, for use in the treatment of migraine.

11. A pharmaceutical composition for treating migraine comprising an amount of 0.5 to 100 mg per unit dose of a tropyl or pseudotropyl benzoate derivative having the formula I according to Claim 10 or a pharmaceutically acceptable salt thereof, in admixture or otherwise associated with a pharmacuetically acceptable diluent or carrier.

12. A composition according to claim 11 containing from 3 to 30 mg of said compund per unit dose.

**Claims for the Contracting State AT**

1. A process for preparing a tropyl or pseudotropyl benzoate derivative having the following general formula I

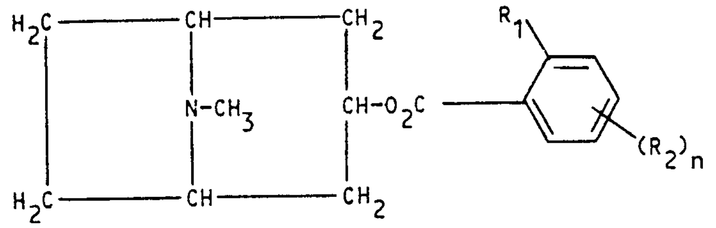

(I)

wherein

$n$ represents 0 or an integer from 1 to 4;

$R_1$ represents $C_1$—$C_4$ alkoxy or chlorine provided that $R_1$ is not chlorine when $n$ is 0;

$R_2$ represents $C_1$—$C_4$ alkyl, $C_1$—$C_4$ alkoxy or halogen, provided that $R_2$ is not chlorine in the 4, 5 or 6 position when $R_1$ is chlorine and $n$ is 1, and that $R_2$ is the same as $R_1$ when $n$ is 2, 3 or 4;

or a pharmaceutically acceptable salt thereof, which comprises reacting tropine or pseudotropine with an acid halide having the formula

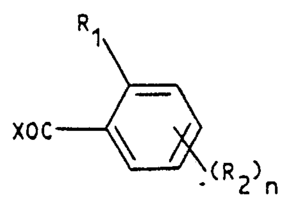

(IV)

wherein $R_1$, $R_2$ and $n$ are as defined above and X represents halogen, and optionally converting the product obtained into a pharmaceutically acceptable salt.

2. A process according to Claim 1 wherein the hydrohalide salt of tropine is heated with the acid halide in the absence of a solvent.

3. A process according to Claim 1 wherein pseudotropine is stirred with the acid halide in an aprotic solvent at ambient temperature.

4. A process according to Claim 1, wherein $R_1$ is methoxy or chlorine and $R_2$ represents methyl, methoxy or chlorine.

5. A process according to Claim 1 wherein $R_1$ and $R_2$ are both chlorine.

6. A process according to Claim 1 wherein $n$ is 1 and the phenyl group is substituted in the 2,3; 2,4 or 2,5 position.

7. The use of a tropyl or pseudotropyl benzoate derivative having the formula I according to Claim 1 or of a pharmaceutically acceptable salt thereof for the preparation of a pharmaceutical composition comprising an amount of 0.5 to 100 mg per unit dose of said compound.

8. Embodiment according to Claim 7 for the preparation of a pharmaceutical composition containing from 3 to 30 mg of said compound per unit dose.

9. The use of a tropyl or pseudotropyl benzoate derivative having the following general formula I

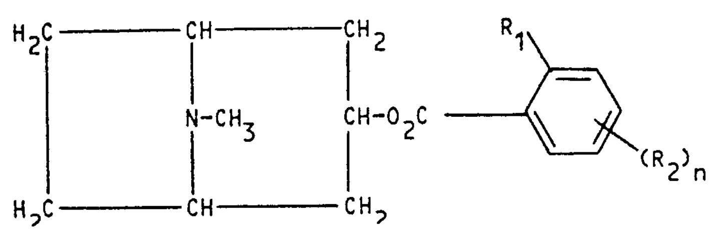

(I)

wherein:

$n$ represents 0 or an integer from 1 to 4;

$R_1$ represents $C_1$—$C_4$ alkoxy or chlorine;

$R_2$ represents $C_1$—$C_4$ alkyl, $C_1$—$C_4$ alkoxy or halogen provided that $R_2$ is the same as $R_1$ when $n$ is 2, 3, or 4;

or a pharmaceutically acceptable salt thereof, for the preparation of a pharmaceutical composition for use in the treatment of migraine.

**Patentansprüche für die Vertragsstaaten BE CH DE FR IT LI LU NL SE**

1. Tropyl- oder Pseudotropyl-Benzoat-Derivat der folgenden allgemeinen Formel I

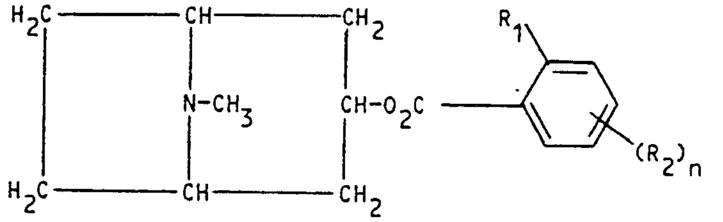

(I)

in der

*n* den Wert 0 hat oder eine ganze Zahl zwischen 1 und 4 bedeutet;

$R_1$ einen $C_1$—$C_4$-Alkoxyrest oder ein Chloratom bedeutet, mit der Maßagbe, daß $R_1$ kein Chloratom ist, wenn *n* den Wert 0 hat;

$R_2$ einen $C_1$—$C_4$-Alkyl-, $C_1$—$C_4$-Alkoxyrest oder Halogen bedeutet, mit der Maßgabe, daß $R_2$ kein Chloratom in 4, 5 oder 6-Stellung ist, wenn $R_1$ ein Chloratom ist und *n* den Wert 1 hat, und daß $R_2$ dieselbe Bedeutung hat wie $4_1$, wenn *n* den Wert 2, 3 oder 4 hat;

oder ein pharmazeutisch verträgliches Salz davon.

2. Verbindung nach Anspruch 1, in der $R_1$ eine Methoxygruppe oder ein Chloratom ist und $R_2$ eine Methyl-, Methoxygruppe oder ein Chloratom bedeutet.

3. Verbindung nach Anspruch 1, in der $R_1$ und $R_2$ ein Chloratom sind.

4. Verbindung nach Anspruch 1, in der *n* den Wert 1 hat und die Phenylgruppe in der 2,3; 2,4 oder 2,5-Stellung substituiert ist.

5. Verfahren zur Herstellung einer Verbindung nach Anspruch 1, bei dem man Tropin oder Pseudotropin mit einem Säurehalogenid der Formel IV

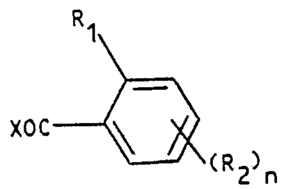

(IV)

umsetzt, in der $R_1$, $R_2$ und *n* die in Anspruch 1 angegebene Bedeutung haben und X Halogen bedeutet.

6. Verfahren nach Anspruch 5, in dem das Halogenwasserstoffsalz von Tropin mit dem Säurehalogenid in Abwesenheit eines Lösungsmittels erhitzt wird.

7. Verfahren nach Anspruch 5, in dem Pseudotropin mit dem Säurehalogenid in einem aprotischen Lösungsmittel bei Umgebungstemperatur gerührt wird.

8. Arzneimittel, enthaltend eine Menge von 0,5 biz 100 mg pro Dosierungseinheit eines Tropyl- oder Pseudotropyl-Benzoat-Derivats der Formel I nach Anspruch 1 oder ein pharmazeutisch verträgliches Salz davon, im Gemisch oder in einer anderen Verbindung mit einem pharmazeutisch verträglichen Verdünnungsmittel oder Träger.

9. Arzneimittel nach Anspruch 8, enthaltend 3 bis 30 mg der Verbindung pro Dosierungseinheit.

10. Tropyl- oder Pseudotropyl-Benzoat-Derivat der folgenden allgemeinen Formel I

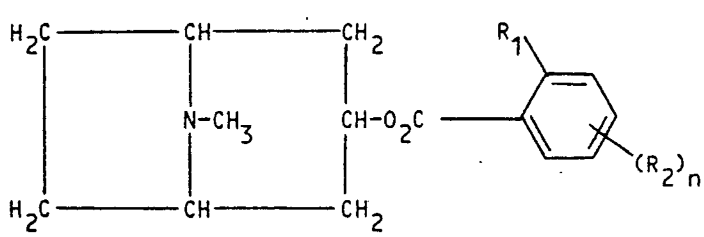

(I)

in der *n* den Wert 0 hat oder eine ganze Zahl von 1 bis 4 ist;

$R_1$ einen $C_1$—$C_4$-Alkoxyrest oder ein Chloratom bedeutet;

$R_2$ einen $C_1$—$C_4$-Alkyl-, $C_1$—$C_4$-Alkoxyrest oder ein Halogen bedeutet, mit der Maßgabe, daß $R_2$ dieselbe Bedeutung wie $R_1$ hat, wenn *n* den Wert 2, 3, oder 4 hat;

oder ein pharmazeutisch verträgliches Salz davon, zur Verwendung bei der Behandlung von Migräne.

11. Arzneimittel zur Behandlung von Migräne, enthaltend eine Menge von 0,5 bis 100 mg pro Dosierungseinheit eines Tropyl- oder Pseudotropyl-Benzoat-Derivats der Formel I nach Anspruch 10 oder eines pharmazeutisch verträglichen Salzes davon, im Gemisch oder in einer anderen Verbindung mit einem pharmazeutisch verträglichen Verdünnungsmittel oder Träger.

12. Arzneimittel nach Anspruch 11, enthaltend 3 bis 30 mg der Verbindung pro Dosierungseinheit.

**Patentansprüche für den Vertragsstaat AT**

1. Verfahren zur Herstellung eines Tropyl- oder Pseudotropyl-Benzoat-Derivats der folgenden allgemeinen Formel I

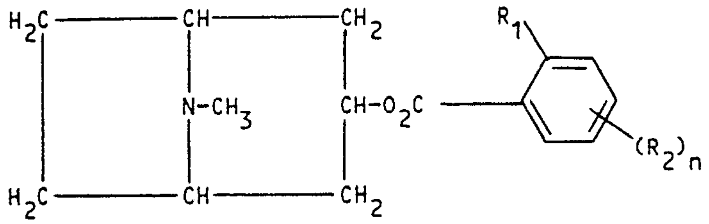

(I)

in der $n$ den Wert 0 hat oder eine ganze Zahl von 1 bis 4 bedeutet;

$R_1$ einen $C_1$—$C_4$-Alkoxyrest oder ein Chloratom bedeutet, mit der Maßgabe, daß $R_1$ kein Chloratom ist, wenn $n$ den Wert 0 hat;

$R_2$ einen $C_1$—$C_4$-Alkyl-, $C_1$—$C_4$-Alkoxyrest oder Halogen bedeutet, mit der Maßgabe, daß $R_2$ kein Chloratom in der 4, 5 oder 6-Stellung ist, wenn $R_1$ ein Chloratom ist und $n$ Wert 1 hat, und daß $R_2$ dieselbe Bedeutung wie $R_1$ hat, wenn $n$ den Wert 2, 3 oder 4 hat;

oder eines pharmazeutisch verträglichen Salzes davon,

bei dem man ein Tropin- oder Pseudotropin mit einem Säurehalogenid der Formel

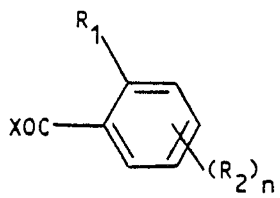

(IV)

umsetzt, in der $R_1$, $R_2$ und $n$ die vorstehend angegebene Bedeutung haben und X ein Halogen bedeutet, und gegebenenfalls das erhaltene Produkt in ein pharmazeutisch verträgliches Salz überführt.

2. Verfahren nach Anspruch 1, in dem das Halogenwasserstoffsalz von Tropin mit dem Säurehalogenid in Abwesenheit eines Lösungsmittels erhitzt wird.

3. Verfahren nach Anspruch 1, im dem Pseudotropin mit dem Säurehalogenid in einem aprotischen Lösungsmittel bei Umgebungstemperatur gerührt wird.

4. Verfahren nach Anspruch 1, in dem $R_1$ eine Methoxygruppe oder ein Chloratom ist und $R_2$ eine Methyl-, Methoxygruppe oder ein Chloratom ist.

5. Verfahren nach Anspruch 1, in dem $R_1$ und $R_2$ ein Chloratom sind.

6. Verfahren nach Anspruch 1, in dem $n$ den Wert 1 hat und die Phenylgruppe in der 2,3; 2,4 oder 2,5-Stellung substituiert ist.

7. Verwendung eines Tropyl- oder Pseudotropyl-Benzoat-Derivats der Formel 1 nach Anspruch 1 oder eines pharmazeutisch verträglichen Salzes davon zur Herstellung eines Arzneimittels, enthaltend eine Menge von 0,5 bis 100 mg pro Dosierungseinheit der Verbindung.

8. Ausführungsform nach Anspruch 7 zur Herstellung eines Arzneimittels, enthaltend 3 bis 30 mg der Verbindung pro Dosierungseinheit.

9. Verwendung eines Tropyl- oder Pseudotropyl-Benzoat-Derivats der folgenden allgemeinen Formel I

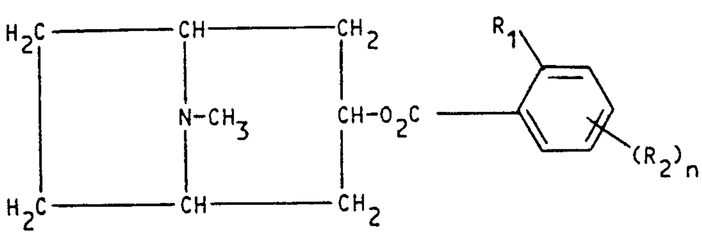

(I)

in der

- $n$ den Wert 0 hat oder eine ganze Zahl zwischen 1 und 4 bedeutet;

$R_1$ einen $C_1$—$C_4$-Alkoxyrest oder ein Chloratom bedeutet;

$R_2$ einen $C_1$—$C_4$-Alkyl-, $C_1$—$C_4$-Alkoxyrest oder ein Halogen bedeutet, mit der Maßgabe, daß $R_2$ dieselbe Bedeutung wie $R_1$ hat, wenn $n$ den Wert 2, 3 oder 4 hat;

oder eines pharmazeutisch verträglichen Salzes davon zur Herstellung eines Arzneimittels zur Verwendung bei der Behandlung von Migräne.

**Revendications pour les Etats Contractants: BE CH DE FR IT LI LU NL SE**

1. Un dérivé de benzoate de tropyle ou de pseudotropyle répondant à la formule générale suivante:

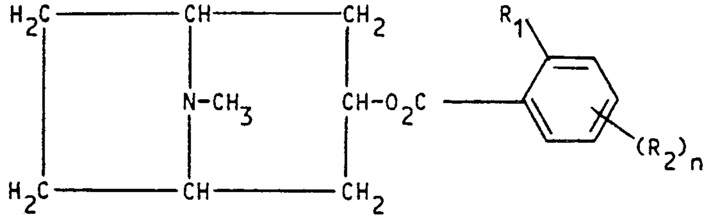

(I)

dans laquelle

*n* représente 0 ou un entier de 1 à 4;

$R_1$ représente un groupe alcoxy en $C_1$—$C_4$ ou un atome de chlore pourvu que $R_1$ ne soit pas le chlore lorsque *n* est égal à 0;

$R_2$ représente un groupe alklye en $C_1$—$C_4$ ou alcoxy en $C_1$—$C_4$ ou un atome d'halogéne, pourvu que $R_2$ ne soit pas le chlore en position 4, 5 ou 6 lorsque $R_1$ est le chlore et *n* est égal à 1, et que $R_2$ soit le même que $R_1$ lorsque *n* est égal à 2, 3, ou 4;

ou un de leurs sels acceptables en pharmacie.

2. Un composé selon la revendication 1, dans lequel $R_1$ est un groupe méthoxy ou un atome de chlore et $R_2$ représente un groupe méthyle ou méthoxy ou un atome de chlore.

3. Un composé selon la revendication 1, dans lequel $R_1$ et $R_2$ sont tous deux des atomes de chlore.

4. Un composé selon la revendication 1, dans lequel *n* est égal à 1 et le groupe phényle est substitué en positions 2,3, 2,4 ou 2,5.

5. Un procédé pour fabriquer un composé selon la revendication 1, qui consiste à faire réagir la tropine ou la pseudotropine avec un halogénure d'acide de formule IV

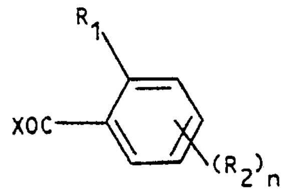

(IV)

dans laquelle $R_1$, $R_2$ et *n* sont tels que définis à la revendication 1 et X représente un halogène.

6. Un procédé selon la revendication 5, dans lequel on chauffe l'halohydrate de tropine avec l'halogénure d'acide en l'absence de solvant.

7. Un procédé selon la revendication 5, dans lequel on agite la pseudotropine avec l'halogénure d'acide dans un solvant aprotique à la température ambiante.

8. Une composition pharmaceutique comprenant une quantité de 0,5 à 100 mg par dose unitaire d'un dérivé de benzoate de tropyle ou de pseudotropyle de formule I selon la revendication 1, ou d'un de ses sels acceptables en pharmacie, en mélange ou autre association avec un diluant ou support acceptable en pharmacie.

9. Une composition selon la revendication 8, contenant de 3 à 30 mg dudit composé par dose unitaire.

10. Un dérivé de benzoate de tropyle ou de pseudotropyle de formule générale I suivante:

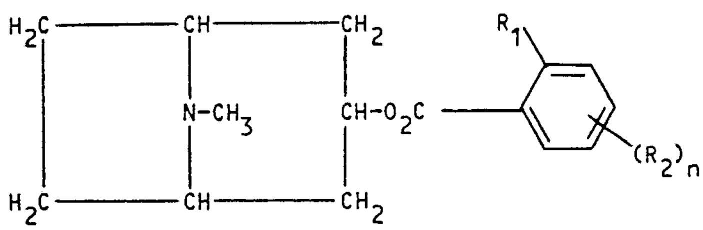

(I)

dans laquelle

*n* représente 0 ou un entier de 1 à 4;

$R_1$ représente un groupe alcoxy en $C_1$—$C_4$ ou un atome de chlore;

$R_2$ représente un groupe alkyle en $C_1$—$C_4$ ou alcoxy en $C_1$—$C_4$ ou un atome d'halogène, pourvu que $R_2$ soit le même que $R_1$ lorsque *n* est 2, 3, ou 4;

ou un de ses sels acceptables en pharmacie, pour l'utilisation dans le traitement de la migraine.

11. Une composition pharmaceutique pour le traitement de la migraine, comprenant une quantité de 0,5 à 100 mg par dose unitaire d'un dérivé de benzoate de tropyle ou de pseudotropyle de formule I selon la revendication 10 ou d'un de ses sels acceptables en pharmacie, en mélange ou autre association avec un diluant ou support acceptable en pharmacie.

12. Une composition selon la revendication 11, contenant de 3 à 30 mg dudit composé par dose unitaire.

**Revendications pour l'Etat Contractant: AT**

1. Une procédé pour fabriquer un dérivé de benzoate de tropyle ou de pseudotropyle répondant à la formule générale suivante

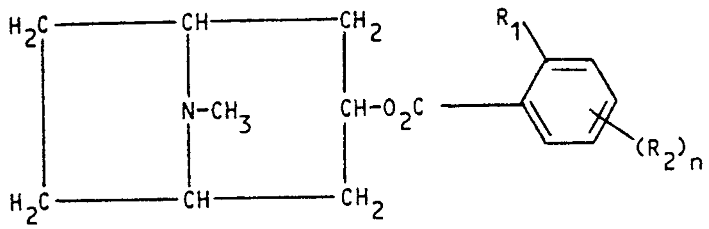

(I)

dans laquelle:

*n* représente 0 ou un entier de 1 à 4;

$R_1$ représente un groupe alcoxy en $C_1$—$C_4$ ou un atome de chlore, pourvu que $R_1$ ne soit pas le chlore lorsque *n* est égal à 0;

$R_2$ représente un groupe alkyle en $C_1$—$C_4$ ou alcoxy en $C_1$—$C_4$ ou un atome d'halogène, pourvu que $R_2$ ne soit pas le chlore en position 4, 5 ou 6 lorsque $R_1$ est le chlore et *n* est égal à 1, et que $R_2$ soit le même que $R_1$ lorsque *n* est égal à 2, 3, ou 4;

ou un de ses sels acceptables en pharmacie, qui consiste à faire réagir la tropine ou la pseudotropine avec un halogénure d'acide de formule IV

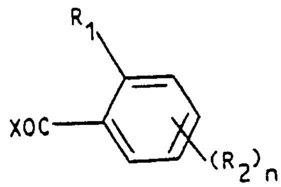

(IV)

dans laquelle $R_1$, $R_2$ et *n* sont tels que définis ci-dessus et X représente un halogène, et à transformer facultativement le produit obtenu en un sel acceptable en pharmacie.

2. Un procédé selon la revendication 1, dans lequel on chauffe l'halohydrate de tropine avec l'halogénure d'acide en l'absence de solvant.

3. Un procédé selon la revendication 1, dans lequel on agite la pseudotropine avec l'halogénure d'acide dans un solvant aprotique à la température ambiante.

4. Un procédé selon la revendication 1, dans lequel $R_1$ est un groupe méthoxy ou un atome de chlore et $R_2$ représente un groupe méthyle ou méthoxy ou un atome de chlore.

5. Un composé selon la revendication 1, dans lequel $R_1$ et $R_2$ sont tous deux des atomes de chlore.

6. Un composé selon la revendication 1, dans lequel *n* est égal à 1 et le groupe phényle est substitué en positions 2,3; 2,4 ou 2,5.

7. L'utilisation d'un dérivé de benzoate de tropyle ou de pseudotropyle de formule I selon la revendication 1 ou d'un de ses sels acceptables en pharmacie pour la préparation d'une composition pharmaceutique contenant 0,5 à 100 mg dudit composé par dose unitaire.

8. Mode de mise en oeuvre selon la revendication 7, pour la préparation d'une composition pharmaceutique contenant de 3 à 30 mg dudit composé par dose unitaire.

9. L'utilisation d'une dérivé de benzoate de tropyle ou de pseudotropyle de formule générale I suivante.

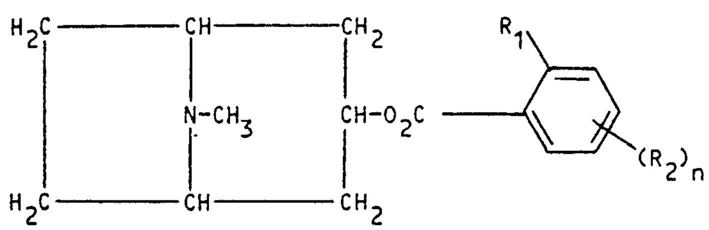

(I)

dans laquelle

*n* représente 0 ou un entier de 1 à 4;

$R_1$ représente un groupe alcoxy en $C_1$—$C_4$ ou un atome de chlore;

$R_2$ représente un groupe alkyle en $C_1$—$C_4$ ou alcoxy en $C_1$—$C_4$ ou un atome d'halogène, pourvu que $R_2$ soit le même que $R_1$ lorsque *n* est 2, 3 ou 4;

ou d'un de ses sels acceptables en pharmacie, pour la préparation d'une composition pharmaceutique pour l'utilisation dans le traitement de la migraine.